(19) Europäisches Patentamt
European Patent Office
Office européen des brevets

(11) Veröffentlichungsnummer: **0 327 875 B1**

(12) **EUROPÄISCHE PATENTSCHRIFT**

(45) Veröffentlichungstag der Patentschrift: **24.03.93**

(51) Int. Cl.[5]: **C07C 209/18**, C07C 211/48

(21) Anmeldenummer: **89101170.2**

(22) Anmeldetag: **24.01.89**

Die Akte enthält technische Angaben, die nach dem Eingang der Anmeldung eingereicht wurden und die nicht in dieser Patentschrift enthalten sind.

# (54) Verfahren zur Herstellung von N-alkylierten Anilinen.

(30) Priorität: **06.02.88 DE 3803661**

(43) Veröffentlichungstag der Anmeldung: **16.08.89 Patentblatt 89/33**

(45) Bekanntmachung des Hinweises auf die Patenterteilung: **24.03.93 Patentblatt 93/12**

(84) Benannte Vertragsstaaten: **BE DE FR GB IT**

(56) Entgegenhaltungen:

**PATENT ABSTRACTS OF JAPAN, Band 12, Nr. 47 (C-475)(2894), 12. Februar 1988; & JP-A-62 195 350**

**CHEMICAL ABSTRACTS, Band 108, Nr. 3, 18. Januar 1988, Seite 558, Spalte 2, Zusammenfassung Nr. 21483y, Columbus, Ohio, USA; & JP-A-62 195 350**

**CHEMICAL ABSTRACTS, Band 84, Nr. 17, 26. April 1976, Seite 500, Spalte 1, Zusammenfassung Nr. 121339k, Columbus, Ohio, USA; T. NOBUO et al: "N-Methylation of aniline with methanol over transition metal zeolite"**

(73) Patentinhaber: **BAYER AG**

**W-5090 Leverkusen 1 Bayerwerk(DE)**

(72) Erfinder: **Klug, Günther, Dr.**
**Wiener-Neustädter-Strasse 140**
**W-4019 Monheim 2(DE)**
Erfinder: **Buysch, Hans-Josef, Dr.**
**Brandenburger Strasse 28**
**W-4150 Krefeld(DE)**
Erfinder: **Puppe, Lothar, Dr.**
**Am Weiher 10 A**
**W-5093 Burscheid(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

CHEMICAL ABSTRACTS, Band 103, Nr. 17, 28. Oktober 1985, Seite 684, Spalte 1, Zusammenfassung Nr. 141538p, Columbus, Ohio, USA; O. MAKOTO et al: "Selective N-monoalkylation of aniline derivatives by use of alkali cation exchanged X- and Y-type zeolites"

CHEMICAL ABSTRACTS, Band 103, Nr. 2, 15. Juli 1985, Seite 75, Spalte 1, Zusammenfassung Nr. 7725u, Columbus, Ohio, USA; G. O. CHIVADZE et al: "Alkylation of aniline by methanol on modified synthetic zeolites"

CHEMICAL ABSTRACTS, Band 107, Nr. 1, 6. Juli 1987, Seite 616, Spalte 1, Zusammenfassung Nr. 6705h, Columbus, Ohio, USA; P. Y. CHEN et al: "The selective alkylation of aniline with methanol over ZSM-5 zeolite"

CHEMICAL ABSTRACTS, Band 109, Nr. 10, 5. September 1988, Seite 127, Spalte 1, Zusammenfassung Nr. 75635p, Columbus, Ohio, USA; K. WU et al: "Alkylation of aniline with methanol over ZSM-5 and ADHM zeolites"

7th Intern. Zeolite Conf., S. 739-44, Tokyo, 1986

## Beschreibung

Die vorliegende Erfindung betrifft ein Verfahren zur Herstellung von N-alkylierten Anilinen durch Umsetzung von Anilinen mit niederen Alkoholen oder Dialkylethern bei erhöhter Temperatur und in Gegenwart von Protonenhaltigen Zeolithen des Pentasil-Typs mit einem $SiO_2/Al_2O_3$-Verhältnis von mindestens 70, die mit einem Bindemittel verpreßt sind.

N-alkylierte Aniline sind wichtige industrielle Zwischenprodukte zur Herstellung von Farbstoffen, Stabilisatoren, Urethanen, Harnstoffen, Pharmaka und Pflanzenschutzmitteln. Sie werden in der Regel durch Alkylierung von Anilinen mit Alkoholen in Gegenwart saurer Katalysatoren, beispielsweise Phosphoroxychlorid, unter Druck oder durch gemeinsames Durchleiten von Anilin- und Alkoholdampf durch heiße Phosphorsäure bei Normaldruck hergestellt.

Diese Verfahren sind in mancher Hinsicht nicht befriedigend. Die Umsetzung in Autoklaven unter Druck ist technisch aufwendig, und durch die homogen gelösten sauren Katalysatoren wird starke Korrosion verursacht. Das drucklose Phosphorsäureverfahren weist diesen Nachteil weniger auf, aber die relativ große Menge Phosphorsäure wird im Laufe der Zeit als Katalysator unbrauchbar und muß entsorgt und durch frische ersetzt werden.

Für beide Verfahren gilt, daß sie hinsichtlich der wechselweisen Herstellung von N-Monoalkyl-anilinen und N,N-Dialkyl-anilinen nur eine relativ geringe Flexibilität aufweisen.

Man hat bereits Zeolithe als Katalysatoren für diese Umsetzungen vorgeschlagen und gezeigt, daß man sowohl N-Monoalkyl- als auch N,N-Dialkyl-aniline erhalten kann. Allerdings treten hierbei andere Nachteile auf.

Nach Waseda Daigaku Rikogaku Konkyusho Hokoku 69 (1975), 21-25 (zitiert in C.A. 84 (1976), 121 339 k) sind Y-Zeolithe, sowohl H-Y als auch mit Cu-, Ni-, Co-, Mn-, Zn-, Ca- und Ce-Ionen beladene, geeignet als Katalysatoren für die Umsetzung von Methanol mit Anilin zu N-methylierten Anilinen. Aus der Tabelle 1 auf Seite 23 dieser Literaturstelle läßt sich jedoch entnehmen, daß alle Umsetzungen von Toluidin-Bildung, also von Kernalkylierung, begleitet sind. Die höchste Aktivität (100 % Umsatz) und dabei eine der höchsten Raten (86 %) an N-Methylierung zeigt H-Y, allerdings nur bei einem unwirtschaftlichen Molverhältnis von Methanol:Anilin = 3:1. Die höchste N-Methylierungsrate (92 %) weist Cu-Y auf, wobei allerdings der Umsatz deutlich niedriger (44,9 %) liegt und die Toluidin-Bildung dennoch 3,4 % beträgt.

Außerdem weist die Reaktion an Cu-Y und H-Y eine sehr ungünstige Temperaturabhängigkeit auf, da nur in einem sehr engen Temperaturbereich um 250 °C herum ein brauchbarer Umsatz erzielt wird (vgl. Fig. 1 und 2 auf Seite 22), bei anderen nur wenig veränderten Temperaturen jedoch sowohl Umsatz als auch Ausbeute an N-alkylierten Anilinen abnehmen.

P.Y. Chen, M.C. Chen, H.Y. Chu, N.S. Chang und T.K. Chuang, Proc. 7th Intern. Zeolite Conf. Y. Murakami, A. Iijima und J.W. Ward (Hrsg.), S. 739-744, Kodansha, Tokyo und Elsevier, Amsterdam, Oxford, New York, Tokyo 1986, zeigen in ihrer Untersuchung der N-Methylierung von Anilin mit Methanol an ZSM-5-Zeolithen, daß Kernalkylierung immer zu beobachten ist und sind der Überzeugung, daß sie von aktiven Zentren and der Oberfläche der Zeolithe verursacht wird. Ferner sollen sowohl die basischen als auch die sauren Eigenschaften der Zeolithe für die katalytische Aktivität verantwortlich seien in der Weise, daß bei einer Imprägnierung der Zeolithe mit Metalloxiden die Kernalkylierung zurückgeht. Dennoch findet man selbst unter den günstigsten Bedingungen (Tabelle auf S. 744) mit MgO/H-ZSM 5 noch eine deutliche Kernalkylierung. Außerdem muß eine Temperatur von 350 °C und ein großer Überschuß an Methanol angewandt werden. Dies ist ein Hinweis auf eine sehr geringe Aktivität solcher Zeolithe. Weiterhin ergibt sich mit steigendem $SiO_2/Al_2O_3$-Verhältnis ein fallender Umsatz für Anilin (Fig. 1 auf S. 741). Die Selektivität steigt dabei zwar, aber selbst im günstigsten Fall beträgt die Kernalkylierung immer noch mehrere %-Anteile (Fig. 2 auf S. 742).

Der saure H-ZSM 5 erscheint folgerichtig als der am wenigsten geeignete Zeolith. Er erzeugt nahezu den niedrigsten Anilinumsatz und wird rasch desaktiviert (Fig. 3 auf S. 743) und verursacht außerdem eine der höchsten Kernalkylierungsraten (Tabelle 2 auf S. 743).

Auch in JP-A-62-195305 (1987) werden kristalline Metallsilikate mit Pentasil-Struktur zur N-Alkylierung aromatischer Amine in der Gasphase eingesetzt. Dabei wird als Vorteil die Bildung der monoalkylierten Produkte genannt. Aber auch nach diesem Verfahren entsteht ein Gemisch von mono- und dialkylierten Produkten.

Es wurde nun überraschend gefunden, daß Protonen-haltige Zeolithe vom Pentasil-Typ mit einem $SiO_2/Al_2O_3$-Verhältnis von mindestens 60, die mit Bindemitteln verpreßt wurden, wesentlich bessere Katalysatoren für die N-Alkylierung von Anilin mit Alkoholen darstellen, nur geringfügige oder praktisch keine Kernalkylierung verursachen, verbesserte Standzeiten aufweisen und auch bei niedrigem Alkohol/Anilin-Verhältnis gute Umsätze erzielen.

Die Erfindung betrifft daher ein Verfahren zur Herstellung von N-alkylierten Anilinen der Formel

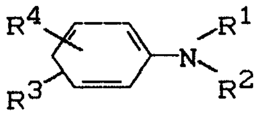
(I)

durch Umsetzung von Anilinen der Formel

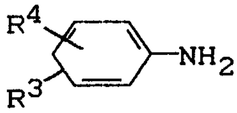
(II),

wobei in den Formeln

| | |
|---|---|
| $R^1$ | $C_1$-$C_4$-Alkyl und |
| $R^2$ | Wasserstoff oder $C_1$-$C_4$-Alkyl bedeuten und |
| $R^3$ und $R^4$ | unabhängig voneinander für Wasserstoff, $C_1$-$C_4$-Alkyl, $C_1$-$C_4$-Alkoxy, Fluor, Chlor oder Brom stehen |

mit $C_1$-$C_4$-Alkoholen oder den entsprechenden Dialkylethern in Gegenwart von Zeolithen bei Temperaturen von 240-350 ° C, das dadurch gekennzeichnet ist, daß man als Zeolithe solche des Pentasil-Typs verwendet, die Protonen enthalten und ein $SiO_2/Al_2O_3$-Verhältnis von 70-1,500 aufweisen, wobei bis zu 50 Äquivalent-% der Protonen durch Metallkationen ausgetauscht sein können, und die weiterhin mit 15 - 50 Gew.-%, bezogen auf den fertigen Zeolith-Katalysator, eines Bindemittels aus der Gruppe von Tonerden, Aluminiumsilikaten und Aluminiumoxiden, bevorzugt $\gamma$-$Al_2O_3$ und $SiO_2$, verpreßt sind.

Geeignete Aniline für das erfindungsgemäße Verfahren sind solche der Formel (II), beispielsweise Anilin oder die isomeren Toluidine, Fluoraniline, Chloraniline, Bromaniline, Xylidine, Ethylaniline, Isopropylaniline und andere, die sich aus (II) ergeben. Bevorzugt sind solche Aniline in denen $R^{11}$ = $C_1$-$C_2$-Alkyl und $R^{12}$ = Wasserstoff oder $C_1$- $C_2$-Alkyl an die Stelle von $R^1$ und $R^2$ treten. Weiter bevorzugte Aniline sind solche, in denen $R^{13}$ und $R^{14}$ mit der voneinander unabhängigen Bedeutung Wasserstoff, $C_1$- $C_2$-Alkyl, $C_1$-$C_2$-Alkoxy, Fluor oder Chlor, bevorzugt $R^{23}$ und $R^{24}$ mit der voneinander unabhängigen Bedeutung Wasserstoff, $C_1$-$C_2$-Alkyl oder Chlor an die Stelle von $R^3$ und $R^4$ treten.

Geeignete Alkylierungsmittel für das erfindungsgemäße Verfahren sind beispielsweise Dimethylether, Diethylether, Methanol, Ethanol, Propanol, Isopropanol, Butanol oder Isobutanol, bevorzugt Methanol und Ethanol.

Die Reaktionspartner können durch Inertgase wie Stickstoff oder Wasserdampf oder durch inerte Lösungsmittel, wie Kohlenwasserstoffe, z,B, Pentan, Cyclohexan, Methylcyclohexan, Isooctan, Benzol, Toluol, Xylol, Cumol oder Ethylbenzol, verdünnt werden.

Geeignete Zeolithe für das erfindungsgemäße Verfahren sind Pentasile, die Protonen enthalten, und unter die Formel

$$H(M_{m/z})[mAlO_2 \cdot nSiO_2] \cdot qH_2O \qquad (III)$$

fallen, in der

n/m, als $SiO_2/Al_2O_3$-Verhältnis gerechnet, mindestens 70 beträgt,

| | |
|---|---|
| $(M_{m/z})$ | teilweise für die Wasserstoffionen im Pentasil vorhandene Metallkationen darstellt, wobei |
| z | die Wertigkeit solcher Kationen angibt, und |
| q | die Menge der sorbierten Wasserphase angibt. |

Das $SiO_2/Al_2O_3$-Verhältnis beträgt erfindungsgemäß 70 bis 1.500, bevorzugt 80 bis 1.000.

Bevorzugte Pentasile sind beispielsweise ZSM 5, ZSM 11, ZSM 8, ZSM 5/ZSM 11-Intermediates, Zeta 1, Zeta 3, ZBM 10, Ultrasil, Ultrazet, TZ-01, NU-4, NU-5 und AZ-1. Besonders bevorzugt sind Pentasile vom Typ ZSM 5, ZSM 8, ZSM 11 und ZSM 5/ZSM 11-Intermediates. Ganz besonders bevorzugt sind Pentasile vom ZSM 5-Typ. Die Herstellung solcher Pentasil-Typen ist bekannt. Hierzu kann verwiesen werden auf D.W. Breck: Zeolite Molecular Sieves, John Wiley and Sons Inc., New York 1974, Russ. J. Phys. Chem. 55 (1981), 1175 und die Patentschriften EP 18 090, EP 34 727, EP 54 386, EP 57 016, EP 65 401, EP 113

116, DE-OS 2 548 697, DE-OS 2 643 929, US 3.702.886, US 3.709.979 und GB 1.334.243.

Die für das erfindungsgemäße Verfahren geeigneten Pentasil-Zeolithe können als Kationen ausschließlich Protonen enthalten. Bis zu 50 Äquivalent-% der Protonen können jedoch auch durch andere Ionen substituiert werden. Hierzu geeignet sind beispielsweise die Ionen von Natrium, Kalium, Magnesium, Zink, Kobalt, Kupfer, Calcium, Eisen, den seltenen Erden (beispielsweise Cer, Lanthan), Zinn, Mangan, Chrom, Titan, Zirkon, Tantal und anderen. In bevorzugter Weise sind in den Pentasilen höchstens 25 Äquivalent-%, in besonders bevorzugter Weise höchstens 10 Äquivalent-% und in der am stärksten bevorzugten Weise höchstens 5 Äquivalent-%, der Protonen durch andere der genannten Metallkationen substituiert.

Die Reaktionsführung des erfindungsgemäßen Verfahrens kann absatzweise unter Rühren in flüssiger Phase und dem sich einstellenden Druck durchgeführt werden, wobei die Pentasil-Zeolithe in stückiger oder in pulveriger Form verwendet werden können. Die Menge des Pentasils beträgt 2 bis 50 Gew.-%, bevorzugt 5 bis 40 Gew.-%, besonders bevorzugt 7 bis 30 Gew.-%, bezogen auf das Gesamtgewicht des Ansatzes.

Die Reaktionsführung kann weiterhin kontinuierlich in der Gasphase durchgeführt werden. Hierbei ist insbesondere eine Durchführung unter Normaldruck möglich. Eine solche kontinuierliche Durchführung ist für die industrielle Anwendung bevorzugt. Hierzu wird ein gegebenenfalls mit Inertgas oder Inertdampf verdünntes Anilin/Alkohol-Dampfgemisch über ein Katalysatorbett von in gekörnter Form vorliegendem Pentasil-Zeolith geleitet. Die Katalysatorbelastung ist hierbei in weiten Grenzen variabel. Gute Umsätze wurden erhalten, wenn eine Katalysatorbelastung im Bereich von 0,01 bis 8,0, bevorzugt 0,05 bis 5,0, besonders bevorzugt 0,1 bis 4,0 Liter umzusetzendes Gemisch/l Katalysator/h eingestellt wird.

Damit die Pentasil-Zeolithe in die für den Betrieb eines Gasphasen-Reaktors günstige stückige Form gebracht werden können, werden sie mit Bindemitteln verpreßt und granuliert. Als Bindemittel geeignet sind verschiedene Tonerden, Aluminiumsilikate und Aluminiumoxide, besonders $\gamma$-$Al_2O_3$ und $SiO_2$. Diese Bindemittel werden in Mengen von 15 bis 50 Gew.-%, bezogen auf den fertigen Zeolith-Katalysator, eingesetzt.

Die Reaktionstemperatur liegt bei 240 bis 350 °C, bevorzugt bei 260 bis 330 °C.

Das Verhältnis des Anilins zum Alkohol ist weitgehend variabel. Jedoch ist es auch dafür verantwortlich, welches Verhältnis an N-monoalkylierten zu N,N-dialkylierten Anilinen im Reaktionsprodukt erhalten wird. So kann im allgemeinen eine Menge von 0,5 bis 3 Mol Alkohol/Mol Anilin eingesetzt werden, bevorzugt 0,7 bis 2 Mol. Wird ein hoher Anteil an N-monoalkyliertem Anilin gewünscht, ist es zweckmäßig, mit einem Molverhältnis Alkohol:Anilin von 0,5:1,2, bevorzugt 0,7:1,0, zu arbeiten.

## Beispiel 1

In ein Reaktionsrohr von ca. 20 mm Durchmesser wurden 20 g eines Zeolithgranulates mit einem mittleren Korndurchmesser von 1 bis 2 mm eingefüllt. Ein Gemisch von Anilin- und Methanoldampf wurde mit unterschiedlichen Katalysatorbelastungen bei verschiedenen Temperaturen mehrere Stunden über dieses Zeolithgranulat geleitet. Die genauen Bedingungen und die Ergebnisse dieser Experimente sind in Tabelle I gesammelt.

Entsprechend den Beispielen 1.1 und 1.2 konnte eine selektive N-Methylierung auch in der Gasphasenreaktion unter Normaldruck erzielt werden. Beispiel 1.3 ergab auch nach 35 Stunden Laufzeit keine Änderung der Aktivität des H-ZSM 5-Zeoliths. Selbst bei extrem hohem $SiO_2/Al_2O_3$-Verhältnis erhielt man noch eine hohe Selektivität bezüglich der N-Alkylierung (Beispiel 1.4). Durch ein höheres Anilinangebot wurde die Selektivität bezüglich der N-Monoalkylierung deutlich verbessert (Beispiel 1.5), womit die Flexibilität des erfindungsgemäßen Verfahrens erwiesen wurde. Nach Vergleichsbeispiel 1.6 wurde unter vergleichbaren Bedingungen mit einem nicht sauren Na-ZSM 5 zwar eine gute Selektivität der N-Methylierung erzielt, aber nur ein völlig unzureichender Umsatz. Bei höheren Temperaturen wurde entsprechend dem obengenannten Stand der Technik zwar der Umsatz gesteigert, aber zugleich auch der Anteil der Kernalkylierung.

Tabelle I

| Nr. | Katalysator | $SiO_2/Al_2O_3$ | Molverh. A/MeOH | Temp. °C | Belast. ml/ml/h | Laufz. h | Produktverteilung Gew.-% A | NMA | NNDMA | kernalk. A |
|---|---|---|---|---|---|---|---|---|---|---|
| 1.1 | H-ZSM 5 + 30 % γ-$Al_2O_3$ | 107 | 1:1 | 300 | 0,5 | 5 | 30 | 40 | 29 | <0,4 |
| 1.2 | " | 107 | 1:1 | 280 | 0,5 | 5 | 40 | 36 | 24 | <0,1 |
| 1.3 | H-ZSM 5 + 15 % $SiO_2$ | 107 | 1:1 | 290 | 0,6 | 35 | 46 | 34 | 10 | 0 |
| 1.4 | H-ZSM 5 + 30 % γ-$Al_2O_3$ | 800 | 1:1 | 300 | 1,0 | 4 | 48 | 32 | 19 | <1 |
| 1.5 | H-ZSM 5 + 30 % γ-$Al_2O_3$ | 107 | 2:1 | 300 | 1,0 | 7 | 60 | 31 | 9 | 0 |
| 1.6 | Na-ZSM 5 + 30 % γ-$Al_2O_3$ | 107 | 1:1 | 300 | 1,0 | 3 | 85 | 10 | 5 | <0,3 |

Beispiel 2

Es wurde so verfahren, wie in Beispiel 1 beschrieben. Als Alkylierungsmittel wurde hier jedoch Ethanol verwendet und zwar im Molverhältnis Anilin:Ethanol = 1:1. Alle Reaktionen wurden bei 300 °C und einer

Katalysatorbelastung von 1,0 ml/ml/h durchgeführt (siehe Tabelle II).

Mit Ethanol erhielt man ebenfalls eine hohe Selektivität in der N-Alkylierung (2.1 und 2.2). Die N-Monoalkylierung fiel hier unter gleichen Bedingungen höher aus als bei der N-Methylierung. Das als Bindermaterial benutzte $\gamma$-Aluminiumoxid allein weist unter den Reaktionsbedingungen einen zu geringen Umsatz, Kernalkylierung und rasche Desaktivierung auf (Vergleichsbeispiel 3.3).

Beispiel 3

Unter den Bedingungen des Beispiels 2 wurde m-Toluidin mit Ethanol umgesetzt.

Mit H-ZSM 5 wurde die erwartete, praktisch 100 %ige N-Alkylierung gefunden und nach 100 Stunden kein Nachlassen der Katalysatoraktivität (3.1).

Im Vergleichsbeispiel 3.2 mit H-Y wurde ein beachtlicher Teil des m-Toluidins im Kern alkyliert.

Tabelle II

| Nr. | Katalysator | $SiO_2/Al_2O_3$ | Laufzeit | Produktverteilung Gew.-% | | | |
|---|---|---|---|---|---|---|---|
| | | | | A | NEA | NNDEA | kernalk. A |
| 2.1 | H-ZSM 5 + 15 % $SiO_2$ | 107 | 4 | 48 | 48 | 4 | 0,1 |
| 2.2 | H-ZSM 5 + 30 % $\gamma$-$Al_2O_3$ | 107 | 4 | 41 | 52 | 6 | <1,5 |
| 2.3 | $\gamma$-$Al_2O_3$ | - | 4 | 77 | 20 | - | 3 (rasche Desaktivierung) |

NEA = N-Ethylanilin
NNDEA = N,N-Diethylanilin

Tabelle III

| Nr. | Katalysator | $SiO_2/Al_2O_3$ | Laufzeit h | Belast. ml/ml/h | Produktverteilung Gew.-% mT | NET | NNDET | kernalk. T |
|---|---|---|---|---|---|---|---|---|
| 3.1 | H-ZSM 5 + 30 % $\gamma$-$Al_2O_3$ | 107 | 100 | 1,3 | 43 | 52 | 5 | <0,1 |
| 3.2 | H-Y + 30 % $\gamma$-$Al_2O_3$ | 4,6 | 4 | 1,0 | 34 | 50 | 6 | 10 |

mT = m-Toluidin
NET = N-Ethyl-m-toluidin
NNDET = N,N-Diethyl-m-toluidin

**Patentansprüche**

1. Verfahren zur Herstellung von N-alkylierten Anilinen der Formel

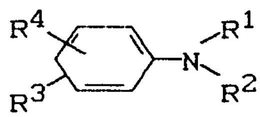

durch Umsetzung von Anilinen der Formel

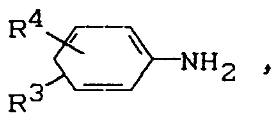

wobei in den Formeln

| | |
|---|---|
| $R^1$ | $C_1$-$C_4$-Alkyl und |
| $R^2$ | Wasserstoff oder $C_1$-$C_4$-Alkyl bedeuten und |
| $R^3$ und $R^4$ | unabhängig voneinander für Wasserstoff, $C_1$-$C_4$-Alkyl, $C_1$-$C_4$-Alkoxy, Fluor, Chlor oder Brom stehen, |

mit $C_1$-$C_4$-Alkoholen oder den entsprechenden Dialkylethern im Verhältnis von 0,5-3 Mol Alkohol/Mol Anilin in Gegenwart von Zeolithen bei Temperaturen von 240-350° C, dadurch gekennzeichnet, daß man als Zeolithe solche des Pentasil-Typs verwendet, die Protonen enthalten und ein $SiO_2/Al_2O_3$-Verhältnis von 70 - 1,500 aufweisen, wobei bis zu 50 Äquivalent-% der Protonen durch Metallkationen ausgetauscht sein können, und die weiterhin mit 15 - 50 Gew.-%, bezogen auf den fertigen Zeolith-Katalysator, eines Bindemittels aus der Gruppe von Tonerden, Aluminiumsilikaten und Aluminiumoxiden, bevorzugt $\gamma$-$Al_2O_3$ und $SiO_2$, verpreßt sind.

2. Verfahren nach Anspruch 1, dadurch gekennzeichnet, daß das $SiO_2/Al_2O_3$-Verhältnis 80 bis 1.000 beträgt.

3. Verfahren nach Anspruch 1, dadurch gekennzeichnet, daß als Pentasil-Typen ZSM 5, ZSM 11, ZSM 8, ZSM 5/ZSM 11-Intermediates, Zeta 1, Zeta 3, ZBM 10, Ultrasil, Ultrazet, TZ-01, NU-4, NU-5 oder AZ-1 eingesetzt werden.

4. Verfahren nach Anspruch 3, dadurch gekennzeichnet, daß als Pentasil-Typen ZSM 5, ZSM 8, ZSM 11 oder ZSM 5/ZSM 11-Intermediates eingesetzt werden.

5. Verfahren nach Anspruch 4, dadurch gekennzeichnet, daß Pentasile vom ZSM 5-Typ eingesetzt werden.

6. Verfahren nach Anspruch 1, dadurch gekennzeichnet, daß 0 bis 50 Äquivalent-% der Protonen gegen Ionen von Natrium, Kalium, Magnesium, Zink, Kobalt, Kupfer, Calcium, Eisen, seltene Erden, Zinn, Mangan, Chrom, Titan, Zirkon und/oder Tantal ausgetauscht sind.

7. Verfahren nach Anspruch 6, dadurch gekennzeichnet, daß 0 bis 25, bevorzugt 0 bis 10, besonders bevorzugt 0 bis 5 Äquivalent-% der Protonen ausgetauscht sind.

8. Verfahren nach Anspruch 1, dadurch gekennzeichnet, daß bei absatzweiser Reaktionsführung in der Flüssigphase 2 bis 50 Gew.-%, bevorzugt 5 bis 40 Gew.-%, besonders bevorzugt 7 bis 30 Gew.-%, Pentasil-Zeolith, bezogen auf das Gesamtgewicht des Ansatzes, angewandt werden.

9. Verfahren nach Anspruch 1, dadurch gekennzeichnet, daß bei bei kontinuierlicher Reaktionsführung in der Gasphase eine Katalysatorbelastung im Bereich von 0,01 bis 8,0, bevorzugt 0,05 bis 5,0, besonders bevorzugt 0,1 bis 4,0 Liter umzusetzendes Gemisch/l Katalysator/h eingestellt wird.

**10.** Verfahren nach Anspruch 1, dadurch gekennzeichnet, daß bei einer Temperatur von 260 bis 330° C, gearbeitet wird.

## Claims

**1.** Process for the preparation of N-alkylated anilines of the formula

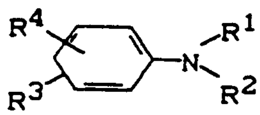

by reaction of anilines of the formula

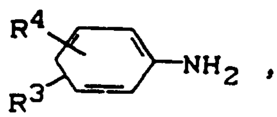

in which formulae

| | |
|---|---|
| $R^1$ | denotes $C_1$-$C_4$-alkyl and |
| $R^2$ | denotes hydrogen or $C_1$-$C_4$-alkyl and |
| $R^3$ and $R^4$ | independently of one another stand for hydrogen, $C_1$-$C_4$-alkyl, $C_1$-$C_4$-alkoxy, fluorine, chlorine or bromine |

with $C_1$-$C_4$-alcohols or the corresponding dialkyl ethers in a ratio of 0.5-3 mol of alcohol/mol of aniline in the presence of zeolites at temperatures of 240-350° C, characterised in that the zeolites used are those of the pentasil type containing protons and having an $SiO_2/Al_2O_3$ ratio of 70-1,500, it being possible to exchange up to 50 equivalent % of the protons for metal cations, and furthermore being compacted with 15-50% by weight, based on the finished zeolite catalyst, of a binder from the group consisting of clays, aluminium silicates and aluminium oxides, preferably $\gamma$-$Al_2O_3$ and $SiO_2$.

**2.** Process according to Claim 1, characterised in that the $SiO_2/Al_2O_3$ ratio is 80 to 1,000.

**3.** Process according to Claim 1, characterised in that the pentasil types used are ZSM 5, ZSM 11, ZSM 8, ZSM 5/ZSM 11-intermediates, zeta 1, zeta 3, ZBM 10, ultrasil, ultrazet, TZ-01, NU-4, NU-5 or AZ-1.

**4.** Process according to Claim 3, characterised in that the pentasil types used are ZSM 5, ZSM 8, ZSM 11 or ZSM 5/ZSM 11-intermediates.

**5.** Process according to Claim 4, characterised in that pentasils of the ZSM 5 type are used.

**6.** Process according to Claim 1, characterised in that 0 to 50 equivalent % of the protons are exchanged for ions of sodium, potassium, magnesium, zinc, cobalt, copper, calcium, iron, rare earths, tin, manganese, chromium, titanium, zirconium and/or tantalum.

**7.** Process according to Claim 6, characterised in that 0 to 25, preferably 0 to 10, particularly preferably 0 to 5, equivalent % of the protons are exchanged.

**8.** Process according to Claim 1, characterised in that, if the reaction is carried out batchwise in the liquid phase, 2 to 50% by weight, preferably 5 to 40% by weight, particularly preferably 7 to 30% by weight, of pentasil zeolite, relative to the total weight of the batch, are used.

**9.** Process according to Claim 1, characterised in that, if the reaction is carried out continuously in the gas phase, the space velocity of the catalyst is adjusted in the range from 0.01 to 8.0, preferably 0.05 to 5.0, particularly preferably 0.1 to 4.0, litres of mixture to be reacted/l of catalyst/h.

**10.** Process according to Claim 1, characterised in that the reaction is carried out at a temperature from 260 to 330 ° C.

**Revendications**

**1.** Procédé de prépararion d'anilines N-alkylées de formule:

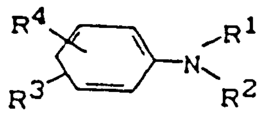

par réaction d'anilines de formule:

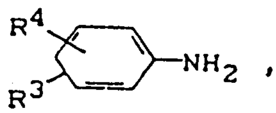

dans lesquelles

$R^1$ représente un groupe alkyle en $C_1$-$C_4$ et

$R^2$ représente l'hydrogène ou un groupe alkyle en $C_1$-$C_4$ et

$R^3$ et $R^4$ représentent chacun, indépendamment l'un de l'autre, l'hydrogène, un groupe alkyle en $C_1$-$C_4$, alcoxy en $C_1$-$C_4$, le fluor, le chlore ou le brome

avec des alcools en $C_1$-$C_4$ ou des éthers dialkyliques correspondants à un rapport de 0,5 à 3 mol d'alcool par mole d'aniline en présence de zéolites, à des températures de 240 à 350 ° C, caractérisé en ce que l'on utilise des zéolites du type pentasil contenant des protons et présentant un rapport $SiO_2/Al_2O_3$ de 70 à 1 500, une proportion allant jusqu'à 50 équivalents % des protons pouvant être remplacée par des cations métalliques, et qui ont en outre été comprimées à l'aide de 15 à 50% en poids, par rapport aux catalyseurs zéolitiques finis, d'un liant du groupe des alumines, des silicates d'aluminium et des oxydes d'aluminium, de préférence à l'aide de $\gamma$-$Al_2O_3$ et de $SiO_2$.

**2.** Procédé selon la revendication 1, caractérisé en ce que le rapport $SiO_2/Al_2O_3$ va de 80 à 1 000.

**3.** Procédé selon la revendication 1, caractérisé en ce que l'on utilise des pentasils des types ZSM 5, ZSM 11, ZSM 8, les produits intermédiaires ZSM 5/ZSM 11, les pentasils Zeta 1, Zeta 3, ZMB 10, Ultrasil, Ultrazet, TZ-01, NU-4, NU-5 ou AZ-1.

**4.** Procédé selon la revendication 3, caractérisé en ce que l'on utilise les pentasils des types ZSM 5, ZSM 8, ZSM 11 ou des types intermédiaires ZSM 5/ZSM 11.

**5.** Procédé selon la revendication 4, caractérisé en ce que l'on utilise des pentasils du type ZSM 5.

**6.** Procédé selon la revendication 1, caractérisé en ce que 0 à 50 équivalents % des protons sont échangés contre des ions sodium, potassium, magnésium, zinc, cobalt, cuivre, calcium, fer, des terres rares, étain, manganèse, chrome, titane, zirconium et/ou tantale.

**7.** Procédé selon la revendication 6, caractérisé en ce que 0 à 25, de préférence 0 à 10, et mieux encore 0 à 5 équivalents % des protons ont été échangés.

**8.** Procédé selon la revendication 1, caractérisé en ce que, à la mise en oeuvre discontinue en phase liquide, on utilise de 2 à 50%, de préférence de 5 à 40%, et mieux encore de 7 à 30% en poids de zéolite du type pentasil par rapport au poids total du mélange de réaction.

**9.** Procédé selon la revendication 1, caractérisé en ce que, à la mise en oeuvre continue en phase gazeuse, on règle la charge du catalyseur dans l'intervalle de 0,01 à 8,0, de préférence de 0,05 à 5,0

et mieux encore de 0,1 à 4,0 l de mélange à convertir par litre de catalyseur et par heure.

**10.** Procédé selon la revendication 1, caractérisé en ce que l'on opère à une température de 260 à 330 °C.